# EUROPÄISCHE PATENTSCHRIFT

(11) **EP 2 700 948 B1**
(45) Veröffentlichungstag und Bekanntmachung des Hinweises auf die Patenterteilung: **30.08.2017**
(21) Anmeldenummer: 13175746.0
(22) Anmeldetag: 09.07.2013
(51) Int. Cl.: G01N 33/537, G01N 33/543

(54) **Verfahren zur Durchführung einer biochemischen Analyse, insbesondere im Weltraum**
Method for performing a biochemical analysis, in particular in space
Procédé de réalisation d'une analyse biochimique, notamment dans l'espace

(30) Priorität: 21.08.2012 DE 102012107651
(43) Veröffentlichungstag der Anmeldung: 26.02.2014
(73) Patentinhaber: Airbus Defence and Space GmbH, 82024 Taufkirchen (DE)
(72) Erfinder: Kern, Peter, 88682 Salem (DE); Backes, Herbert, 66130 Saarbrücken (DE); Kübler, Ullrich, 88677 Markdorf (DE)
(74) Vertreter: Daub, Thomas

(56) Entgegenhaltungen:
- EP-A1- 2 634 579
- US-A- 6 100 079
- US-A1- 2005 250 141
- US-A1- 2006 286 563
- US-A1- 2009 311 724
- US-A1- 2010 248 258
- US-A1- 2012 178 186
- YOUNGEUN KWON ET AL: "Magnetic Bead Based Immunoassay for Autonomous Detection of Toxins", ANALYTICAL CHEMISTRY, Bd. 80, Nr. 22, 15. November 2008 (2008-11-15), Seiten 8416-8423, XP055033445, ISSN: 0003-2700, DOI: 10.1021/ac8010044
- MOSER Y ET AL: "Active superparamagnetic bead manipulation for immunoassays on-chip", THE PROCEEDINGS OF [MICRO]TAS 2008 CONFERENCE : VOLUMES 1 & 2 ; THE TWELFTH INTERNATIONAL CONFERENCE ON MINIATURIZED SYSTEMS FOR CHEMISTRY AND LIFE SCIENCES ; SAN DIEGO SHERATON HOTEL & MARINA, SAN DIEGO, CALIFORNIA, USA, OCTOBER 12 - 16, 2008, CHEMI, 12. Oktober 2008 (2008-10-12), Seiten 1372-1374, XP002680448, ISBN: 978-0-9798064-1-4

## Beschreibung

### Stand der Technik

Die Erfindung betrifft ein Verfahren zur Durchführung einer biochemischen Analyse im Weltraum nach dem Oberbegriff des Anspruchs 1. Eine häufig genutzte biochemische Analysemethode zum qualitativen und/oder quantitativen Nachweis eines Analyten in einer Probe ist von den als Immunoassays bezeichneten Verfahren gebildet. Immunoassays beruhen auf dem Funktionsprinzip einer selektiven Bindung eines Analyten in der Probe durch ein analytspezifisches Paar von Fängerantikörpern (capture-Antikörpern, cAB) und Detektionsantikörpern (detection-Antikörper, dAB), wobei letztere eine Markersubstanz an sich gebunden tragen oder zur Bindung der Markersubstanz im Verlauf des Verfahrens vorgesehen sind. Die Fängerantikörper sind dazu vorgesehen, den Analyten an einer festen Position, beispielsweise eine Oberfläche, an der die Fängerantikörper gebunden sind, oder an Trägerteilchen für die Fängerantikörper, zu fixieren. Der Detektionsantikörper bindet selektiv an den Analyten oder an den Fängerantikörper. Über die Markersubstanz wird ein messbares Signal, das zu einer Ermöglichung einer Detektion eines entstandenen Analytkomplexes aus Analyt, Fängerantikörper und Detektionsantikörper vorgesehen ist, erzeugt. In den als so genannte Enzyme-linked Immunosorbent Assays (ELISA's) bezeichneten Immunoassays wird der Analyt mittels eines Enzyms als Markersubstanz markiert, das an dem Detektionsantikörper fixiert vorliegen oder in einem weiteren Reaktionsschritt an den Detektionsantikörper gebunden wird, wobei in einer nachfolgenden enzymkatalysierten Reaktion eine chromogene oder eine lumineszente Verbindung, beispielsweise eine chemo-, elektro-, biolumineszente oder fluoreszente Verbindung, aus einem zugegebenen Substrat erzeugt wird, die mit optischen Methoden detektiert werden kann. Um eine Sättigung eines Signals der chromogenen oder luminszenten Verbindung zu vermeiden, wird nach einer vorgegebenen Zeitspanne ein Stoppermittel zur Unterbrechung der enzymkatalysierten Reaktion hinzugegeben. Das Stoppermittel kann die Unterbrechung beispielsweise durch eine Veränderung eines pH-Werts bewirken, wobei über die Veränderung des pH-Werts häufig ein entstandenes Produkt aus der Reaktion des Substrats mit dem Enzym in der Art eines pH-Indikators sichtbar gemacht wird. Im Fall der sogenannten Radio-Immunoassays (RIA's) werden radioaktive Substanzen als an den Detektionsantikörper gebundene Markersubstanzen verwendet, wobei eine quantitative Bestimmung des Analyten über eine Messung der Radioaktivität erfolgt. Insbesondere für eine präzise quantitative Bestimmung des Analyten ist eine sorgfältige Durchmischung der Probe, der Fängerantikörper, der Detektionsantikörper und der Markersubstanz nötig. Bei Anwendungen eines Immunoassays auf der Erde kann zu einer Erreichung einer ausreichenden Durchmischung ein diffusiver Stofftransport durch die Wirkung der Erdschwerkraft ausreichen oder ein Reaktionsgefäß wird mechanisch bewegt, beispielsweise gerüttelt. US2012/0178186 beschreibt Bindungsassays zum Nachweis von Analyten in einer Probe, bei denen Magnetpartikel markierte Detektionsantikörper mittels eines äußeren Magnetfelds bewegt werden.

### Vorteile der Erfindung

Die Erfindung geht aus von einem Verfahren zur Durchführung einer biochemischen Analyse im Weltraum unter Bedingungen reduzierter Schwerkraft, bei der zumindest ein Analyt in einer Probe qualitativ und/oder quantitativ mittels einer selektiven Bindung eines analytspezifischen Paars aus einer Bindesubstanz, die den Analyten fixiert, und einer Detektionssubstanz, die an den Analyt bindet und eine Markersubstanz zu einer Quantifizierung des Analyten an sich gebunden trägt oder an sich bindet, bestimmt wird und wobei in einem Verfahrensschritt eine Durchmischung der Probe, der Bindesubstanz, der Detektionssubstanz und der Markersubstanz in einem Reaktionsgefäß erfolgt, wobei das Verfahren in einer das Reaktionsgefäß und eine Magneteinheit umfassenden Vorrichtung durchgeführt wird. Grundsätzlich können bei der Durchmischung der Probe, der Bindesubstanz, der Detektionssubstanz und der Markersubstanz in dem Reaktionsgefäß auch Lösungsmittel und weitere Hilfsstoffe in dem Reaktionsgefäß enthalten sein. Eine solche biochemische Analyse wird als Immunoassay bezeichnet. Unter einer "Durchführung im Weltraum" soll insbesondere verstanden werden, dass die Durchführung der biochemischen Analyse außerhalb der Erde, beispielsweise in einem Raumfahrzeug in einem Erdorbit oder an einem Lagrange-Punkt, während eines Raumflugs oder eines Umlaufs um einen anderen Planeten oder um einen Mond, auf einem Satelliten, einem Mond, einem Asteroiden oder auf einem von der Erde verschiedenen Planeten erfolgt. Insbesondere kann die Durchführung im Weltall unter Bedingungen reduzierter Schwerkraft stattfinden. Unter "Bedingungen reduzierter Schwerkraft" sollen insbesondere Bedingungen verstanden werden, bei denen eine Schwerewirkung von maximal 0,9 g, vorteilhaft maximal 1*10⁻³ g, vorzugsweise maximal 1*10⁻⁶ g und besonders bevorzugt maximal 1*10⁻⁸ g wirksam ist. Mit "g" ist der Wert der Fallbeschleunigung auf der Erde von 9,81 m/s² bezeichnet.

Unter einer "biochemischen Analyse" soll insbesondere eine qualitative und/oder quantitative Analyse einer biochemischen Stoffmischung auf einen Analyten hin verstanden werden. Unter einem "Analyten" soll insbesondere ein chemisches Element oder eine chemische Substanz, beispielsweise eine Molekülklasse oder ein spezifisches Molekül oder Makromolekül wie ein Protein, verstanden werden, die in einer Stoffmischung qualitativ, d.h. lediglich auf ein Vorhandensein, und/oder quantitativ, d. h. auf eine Konzentration und/oder Menge, untersucht werden soll. Unter einer "Probe" soll insbesondere eine zu analysierende Stoffmischung verstanden werden, vorzugsweise eine komplexe biologische Matrix, wie beispielsweise Blut, Blutplasma oder -serum, Urin, Speichel oder andere Lösungen. Unter einer "Bindesubstanz" soll insbesondere eine Substanz verstanden werden, die dazu vorgesehen ist, den Analyten an sich zu binden. Insbesondere kann die Bindesubstanz von einem Protein, vorzugsweise von einem Antikörper, gebildet sein. Unter einer "Detektionssubstanz" soll insbesondere eine Substanz verstanden werden, die an den Analyten oder die Bindesubstanz binden kann und die an sich gebunden die Markersubstanz trägt oder dazu vorgesehen ist, die Markersubstanz an sich zu binden. Insbesondere kann die Detektionssubstanz von einem Protein, vorzugsweise von einem Antikörper, gebildet sein. Unter einer "Markersubstanz" soll insbesondere eine Substanz verstanden werden, die an die Detektionssubstanz gebunden ist oder an diese binden kann und die dazu vorgesehen ist, ein Signal zu einer Quantifizierung des Analyten und zu einer Detektion eines Analytkomplexes aus dem Analyten, der Bindesubstanz und der Detektionssubstanz zu erzeugen. Die Markersubstanz kann beispielsweise als Farbstoff oder als Fluorophor oder als ein Enzym, das eine signalgebende Reaktion, beispielsweise einen Farbumschlag durch eine Substratspaltung, katalysiert, ausgebildet sein. Unter einem "analytspezifischen Paar aus einer Bindesubstanz und einer Detektionssubstanz" soll insbesondere verstanden werden, dass die Bindesubstanz dazu vorgesehen ist, speziell an den Analyten zu binden und die Detektionssubstanz speziell dazu vorgesehen ist, an den Analyten bzw. an die Bindesubstanz zu binden und dass die Bindesubstanz und die Detektionssubstanz vorzugsweise eine geringe Bindungsfähigkeit an weitere Stoffe, besonders an eventuelle weitere Analyten, in der Stoffmischung aufweisen. Unter einem "Reaktionsgefäß" soll insbesondere ein speziell zu einer Durchführung einer Analyse vorgesehenes Gefäß mit einem Arbeitsvolumen, innerhalb dessen die Reaktion stattfindet, verstanden werden.

Es wird vorgeschlagen, dass die Durchmischung mittels Mischungskörpern bewirkt wird, wobei die Mischungskörper dazu vorgesehen sind, eine Durchmischung der Probe, der Bindesubstanz und der Detektionssubstanz zu bewirken, wobei die Mischungskörper durch eine interne Bewegungsvorrichtung oder durch eine externe Kraft in Bewegung versetzt werden und aufgrund ihrer Bewegung die Probe, die Bindesubstanz und die Detektionssubstanz durchmischen. Unter "Mischungskörpern" sollen insbesondere Körper verstanden werden, die speziell dazu vorgesehen sind, eine Durchmischung zumindest zweier verschiedener Stoffe und/oder Stoffmischungen zu einer gemeinsamen Stoffmischung bewirken. Insbesondere werden die Mischungskörper durch eine interne Bewegungsvorrichtung oder durch eine externe Kraft in Bewegung versetzt und durchmischen aufgrund ihrer Bewegung die verschiedenen Stoffe und/oder Stoffmischungen. Es kann insbesondere ein Verfahren mit einer vorteilhaften, schnell und verlässlich durchführbaren Durchmischung und einem raschen Prozessablauf erreicht werden. Ferner kann insbesondere eine rasche Durchmischung auch unter Bedingungen reduzierter Schwerkraft, beispielsweise im Weltraum, erreicht werden, bei denen ein diffusiver Stofftransport nur sehr langsam abläuft. Erfindungsgemäß wird vorgeschlagen, dass die Durchmischung mittels magnetisch bewegter Mischungskörper bewirkt wird und wobei die Mischungskörper als magnetische Inertkörper ausgebildet sind, wobei die magnetischen Inertkörper magnetische oder magnetisierbare Partikel sind, die frei von Bindungsmöglichkeiten zu Stoffen in der Probe und frei von an sie gebundenen analytspezifischen Bindesubstanzen oder analytspezifische Detektionssubstanzen sind. Unter "magnetisch bewegten Mischungskörpern" sollen insbesondere Mischungskörper verstanden werden, die durch ein angelegtes magnetisches Feld, vorzugsweise ein magnetisches Wechselfeld, in Bewegung, insbesondere eine translatorische oder eine rotatorische Bewegung, versetzt werden können. Die Durchmischung wird mittels magnetischer Inertkörper bewirkt. Unter "magnetischen Inertkörpern" sollen insbesondere magnetische oder magnetisierbare Partikel verstanden werden, die speziell dazu ausgebildet sind, frei von Bindungsmöglichkeiten zu Stoffen in der Probe, Bindesubstanzen, Detektionssubstanzen und Markersubstanzen zu sein. Insbesondere sind die magnetischen Inertkörper frei von an sie gebundenen analytspezifischen Bindesubstanzen oder analytspezifische Detektionssubstanzen. Es kann insbesondere eine mit geringem apparativem Aufwand zu realisierende Durchmischung erreicht werden.

Ferner wird vorgeschlagen, dass die Durchmischung mittels magnetischer Trägerkörper für die analytspezifische Bindesubstanz oder für die analytspezifische Detektionssubstanz bewirkt wird. Unter "magnetischen Trägerkörpern" sollen insbesondere magnetische oder magnetisierbare Partikel verstanden werden, die die analytspezifische Bindesubstanz und/oder die Detektionssubstanz an sich gebunden, beispielsweise aufgrund einer kovalenten oder einer adsorptiven Bindung, tragen. Insbesondere sind die magnetischen Trägerkörper dazu vorgesehen, nach einem Abschluss einer chemischen Reaktion der Analyse zu einer Auslesung mittels eines Magnetfelds positioniert zu werden. Es kann insbesondere eine vorteilhafte Funktionsintegration erreicht werden.

Ferner wird vorgeschlagen, dass die Bindesubstanz und/oder die Detektionssubstanz zumindest in einem Verfahrensschritt in einer zumindest weitgehend wasserfreien Form verwendet werden. Unter einer "zumindest weitgehend wasserfreien Form" soll insbesondere eine Form verstanden werden, bei der die Bindesubstanz und/oder die Detektionssubstanz als ein Feststoff vorliegen, wobei innerhalb des Feststoffs ein zu einer Verhinderung einer Schädigung einer Funktion der Bindesubstanz und/oder der Detektionssubstanz ausreichender Anteil an Wasser und/oder einem dazu geeigneten Additiv enthalten sein kann. Insbesondere kann die Bindesubstanz und/oder die Detektionssubstanz in einem weiteren Verfahrensschritt in Lösung gebracht werden. Es kann insbesondere eine einfache und platzsparende Lagerung der Bindesubstanz und/oder der Detektionssubstanz ermöglicht werden.

Ferner wird vorgeschlagen, dass die Bindesubstanz und/oder die Detektionssubstanz in zumindest einem weiteren Verfahrensschritt in Lösung gebracht werden. Insbesondere können die Bindesubstanz und die Detektionssubstanz in getrennten Kammern gelagert werden und durch ein Weiterströmen von Lösungsmitteln miteinander in Kontakt gebracht und in das Reaktionsgefäß eingebracht werden. Alternativ können die Bindesubstanz und die Detektionssubstanz in derselben Kammer gelagert werden und gleichzeitig in Lösung gebracht werden. Es kann insbesondere eine vorteilhafte Durchmischung erreicht werden.

Erfindungsgemäß wird vorgeschlagen, dass eine an einer räumlich vorgegebenen Stelle des Reaktionsgefäßes unablöslich befestigte Bindesubstanz verwendet wird. Unter "unablöslich befestigt" soll insbesondere verstanden werden, dass die Bindesubstanz bei Zugabe weiterer Stoffe während der biochemischen Analyse, insbesondere der Probe und der Detektionssubstanz sowie von Lösungsmitteln, und bei der Durchmischung der weiteren Stoffe an der räumlich vorgegebenen Stelle befestigt bleibt. Insbesondere ist die Bindesubstanz an einer für eine Auslesung eines durch die Markersubstanz erzeugten Signals zur Quantifizierung des Analyten vorteilhaften Position angeordnet. Es kann insbesondere eine einfach durchzuführende Auslesung erreicht werden. Erfindungsgemäß wird vorgeschlagen, dass die Durchmischung unter Bedingungen reduzierter Schwerkraft erfolgt. Es können insbesondere Störbedingungen, wie eine schwerkraftbedingte Sedimentierung von gelagerten Stoffen oder eine Phasentrennung von Stoffen innerhalb einer Stoffmischung, vermindert werden.

Ferner wird vorgeschlagen, dass die Trägerkörper mittels einer Magneteinheit zu einer Auslesung positioniert werden. Unter einer "Auslesung" soll insbesondere eine Detektion und eine quantitative Auswertung zumindest eines Signals, das von der Markersubstanz erzeugt wird, zu einem quantitativen Nachweis des Analyten verstanden werden. Die Auslesung kann zudem beispielsweise mit einer räumlichen Auflösung oder als integraler Meßwert über einen gesamten Detektionsraum erfolgen und/oder mit einer Bildverarbeitung gekoppelt sein. Unter "zu einer Auslesung positioniert" soll insbesondere verstanden werden, dass die Trägerkörper mittels der Magneteinheit zu einer Auslesung, vorzugsweise einer Auslesung mittels optischer Methoden, derart innerhalb eines Reaktionsgefäßes positioniert werden, dass eine Auslesung mit einer hohen Genauigkeit möglich ist. Insbesondere werden die Trägerkörper hierzu flächig innerhalb des Detektionsraums positioniert, so dass sie vorzugsweise eine dünne Lage bilden. Es kann insbesondere eine apparativ einfach zu realisierende Auslesung erreicht werden.

Ferner wird vorgeschlagen, dass eine Positionierung der Mischungskörper zu einer Auslesung mittels einer künstlich erzeugten Schwerkraft bewirkt wird. Insbesondere wird die Durchmischung mittels einer Zentrifugation oder mittels Schütteln und einer anschließenden Sedimentation der Mischungskörper bewirkt. Es kann insbesondere eine apparativ einfach zu realisierende Positionierung erreicht werden.

Ferner wird zumindest ein zweites analytspezifisches Paar von Bindesubstanzen und Detektionssubstanzen, das zu einer Bestimmung eines weiteren Analyten vorgesehen ist, vorgeschlagen. Grundsätzlich kann eine beliebig große Anzahl von analytspezifischen Paaren von Bindesubstanzen und Detektionssubstanzen, die zur Bestimmung weiterer Analyten vorgesehen sind, verwendet werden. Die Detektionssubstanzen können dieselbe Markersubstanz oder unterschiedliche Markersubstanzen an sich gebunden tragen. Es können insbesondere in einer einzelnen Reaktion mehrere Analyten bestimmt und somit Zeit und zusätzliche Gerätschaften eingespart werden.

Ferner wird vorgeschlagen, dass magnetische Trägerkörper für unterschiedliche analytspezifische Bindesubstanzen oder für unterschiedliche analytspezifische Detektionssubstanzen voneinander bei einer Auslesung unterscheidbar ausgebildet sind. Unter "voneinander bei einer Auslesung unterscheidbar ausgebildet" soll insbesondere verstanden werden, dass die Trägerkörper für unterschiedliche analytspezifische Bindesubstanzen oder für unterschiedliche analytspezifische Detektionssubstanzen Meßsignale aussenden, die voneinander verschieden und von einem Signal der Markersubstanz verschieden sind, so dass sie bei einer Auslesung voneinander und von einem Signal der Markersubstanz voneinander unterschieden werden können. Beispielsweise können die Trägerkörper derart ausgebildet sein, dass ein Trägerkörper für eine Bindesubstanz für einen ersten Analyten nach einer Bestrahlung mit Licht ein Fluoreszenzsignal aussendet, dessen Wellenlänge verschieden von der Wellenlänge eines Fluoreszenzsignals eines Trägerkörpers für eine Bindesubstanz für einen zweiten Analyten und eines Fluoreszenzsignals einer Markersubstanz ist, so dass eine aus gemessenen Fluoreszenzsignalen der Trägerkörper der Bindesubstanzen und der Markersubstanz bei einer Auslesung eine quantitative Bestimmung des ersten und des zweiten Analyten möglich ist. Alternativ können die Trägerkörper auch aufgrund unterschiedlicher geometrischer Eigenschaften, beispielsweise eines unterschiedlichen Durchmessers, voneinander bei einer Auslesung unterscheidbar ausgebildet sein. Es kann insbesondere mit einem einzelnen Ausleseschritt eine quantitative Bestimmung mehrerer Analyten vorgenommen und Zeit und apparativer Aufwand eingespart werden.

Ferner wird eine Vorrichtung mit Mischungskörpern, die zur Durchmischung der Probe, der Bindesubstanz und der Detektionssubstanz vorgesehen sind, vorgeschlagen. Es kann somit insbesondere eine sichere Durchmischung, insbesondere unter Mikroschwerkraftbedingungen, erreicht werden.

Ferner wird vorgeschlagen, dass die Mischungskörper als magnetisch bewegbare Mischungskörper ausgebildet sind. Es kann somit insbesondere mit geringem apparativem und konstruktivem Aufwand eine sichere Durchmischung erreicht werden.

Ferner wird vorgeschlagen, dass die Mischungskörper als magnetische Inertkörper ausgebildet sind. Es kann somit eine allgemein einsetzbare und unspezifische Vorrichtung zur Durchführung einer biochemischen Analyse erreicht werden.

Ferner wird vorgeschlagen, dass die Mischungskörper als magnetische Trägerkörper für die analytspezifische Bindesubstanz oder für die analytspezifische Detektionssubstanz ausgebildet sind. Es kann insbesondere eine vorteilhafte Funktionsintegration erreicht werden.

### Zeichnungen

Weitere Vorteile ergeben sich aus der folgenden Zeichnungsbeschreibung. In den Zeichnungen sind fünf Ausführungsbeispiele dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein erstes Ausführungsbeispiel eines Verfahrens zur Durchführung einer biochemischen Analyse, bei der eine Durchmischung mittels magnetischer Inertkörper bewirkt wird,
- Fig. 2: ein weiteres Ausführungsbeispiel eines Verfahrens zur Durchführung einer biochemischen Analyse, bei der zwei Analyten in einer Probe bestimmt werden und eine Durchmischung mittels magnetischer Trägerkörper für Detektionssubstanzen bewirkt wird,
- Fig. 3: ein weiteres Ausführungsbeispiel analog zu Fig. 2, bei dem eine Durchmischung mittels magnetischer Trägerkörper für Bindesubstanzen bewirkt wird,
- Fig. 4: ein erfindungsgemäßes Ausführungsbeispiel eines Verfahrens zur Durchführung einer biochemischen Analyse, bei der zwei ortsgebundene Bindesubstanzen in einem Reaktionsgefäß verwendet werden und eine Durchmischung mittels magnetischer Inertkörper bewirkt wird, und
- Fig. 5: ein weiteres Ausführungsbeispiel analog zu Fig. 3, in dem ein zusätzliches Ladegefäß verwendet wird.

### Beschreibung der Ausführungsbeispiele

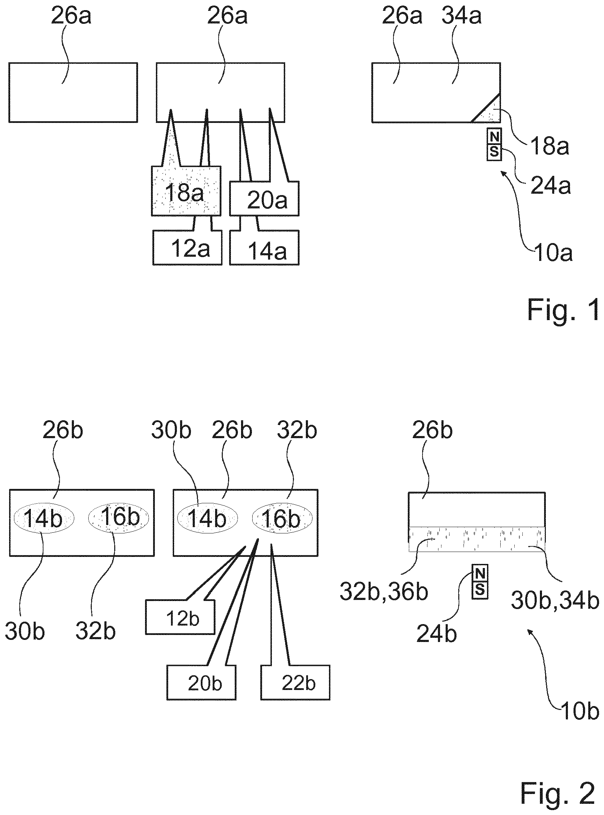

Fig. 1 zeigt eine erste Ausführung eines Verfahrens zur Durchführung einer biochemischen Analyse im Weltraum, bei der ein Analyt in einer Probe 12a qualitativ und quantitativ mittels einer selektiven Bindung eines analytspezifischen Paars aus einer Bindesubstanz 14a, die den Analyten fixiert, und einer Detektionssubstanz 20a, die eine Markersubstanz zu einer Markierung des Analyten an sich gebunden trägt, und wobei in einem Verfahrensschritt eine Durchmischung der Probe 12a, der Bindesubstanz 14a, der Detektionssubstanz 20a und der Markersubstanz in einem Reaktionsgefäß 26a erfolgt. Das Verfahren ist als ein Immunoassay ausgebildet, so dass die Bindesubstanz 14a als ein capture-Antikörper und die Detektionssubstanz 20a als ein detection-Antikörper ausgebildet ist. Der Immunoassay ist ferner als ein Enzyme-linked Immunosorbent Assay (ELISA) ausgebildet, sodass die Markersubstanz als ein Enzym zu einer Spaltung eines im Verlauf des Verfahrens zuzugebenden Substrats, das nach einer Spaltung einen sichtbaren Farbumschlag erzeugt, ausgebildet ist. In alternativen Ausgestaltungen des Verfahrens kann die Markersubstanz beispielsweise als ein Enzym, das ein Substrat zu einer Erzeugung eines Fluoreszenzsignals spaltet, oder als ein Fluorophor oder eine andere geeignete, signalgebende Substanz ausgebildet sein und zusätzlich kann die Markersubstanz separat in das Reaktionsgefäß 26a eingeführt werden und erst im Verlauf des Verfahrens an die Detektionssubstanz 20a binden anstatt zu einem Beginn des Verfahrens an die Detektionssubstanz 20a gebunden zu sein. Das Verfahren wird in einer das Reaktionsgefäß 26a und eine Magneteinheit 24a umfassenden Vorrichtung 10a durchgeführt. Das Reaktionsgefäß 26a ist aus einem transparenten Cyclo-Olefin-Copolymer mit einer vorteilhaft geringen unspezifischen Bindungsfähigkeit hergestellt. Alternativ kann das Reaktionsgefäß 26a auch aus einem anderen Kunststoff, beispielsweise aus Polystyrol, hergestellt sein. Der Kunststoff ist vorzugsweise transparent ausgeführt, sodass eine Auslesung markierter Analyten mittels optischer Methoden erfolgen kann. Alternativ ist durch geeignete Wahl der an die analytspezifische Detektionssubstanz 20a gebundenen Markersubstanz eine Auslesung mittels radiometrischer Methoden möglich. Die Vorrichtung 10a umfasst ferner magnetisch bewegte Mischungskörper, die von magnetischen Inertkörpern 18a gebildet sind. Die magnetischen Inertkörper 18a bestehen aus einem magnetisch polarisierbaren Material, das eine geringe unspezifische Bindungsfähigkeit aufweist und insbesondere eine geringe Bindungsfähigkeit zu dem Analyten in der Probe 12a, der Bindesubstanz 14a, der Detektionssubstanz 20a und der Markersubstanz aufweist.

In einem ersten Verfahrensschritt ist das Reaktionsgefäß 26a vollständig leer. In einem weiteren Verfahrensschritt werden gleichzeitig die magnetischen Inertkörper 18a, die Probe 12a sowie die Bindesubstanz 14a, die Detektionssubstanz 20a und die Markersubstanz zusammen mit weiteren Hilfsstoffen in einer Lösung in das Reaktionsgefäß 26a eingeführt. In alternativen Ausgestaltungen können die magnetischen Inertkörper 18a, die Probe 12a sowie die Bindesubstanz 14a, die Detektionssubstanz 20a und die Hilfsstoffe in mehreren Teilschritten sukzessive in das Reaktionsgefäß 26a eingeführt werden. Die Durchmischung wird mittels magnetisch bewegter Mischungskörper bewirkt. Die Mischungskörper sind von den magnetischen Inertkörpern 18a gebildet. Die Magneteinheit 24a ist als ein Elektromagnet ausgebildet, der mittels eines magnetischen Wechselfelds die magnetischen Inertkörper 18a in Bewegung versetzt, wodurch die Durchmischung der Probe 12a, der Bindesubstanz 14a und der Detektionssubstanz 20a bewirkt wird. Alternativ kann die Magneteinheit 24a auch als beweglich gelagerter Permanentmagnet ausgebildet sein, der zur Erzielung eines örtlich und zeitlich variierenden Magnetfelds innerhalb des Reaktionsgefäßes 26a selbst in Bewegung versetzt wird. Eine Durchführung des Verfahrens und insbesondere die Durchmischung erfolgt unter Bedingungen reduzierter Schwerkraft im All. Das Verfahren kann jedoch auch beispielsweise auf einem Asteroiden, einem Mond oder einem erdfremden Planeten, an Bord eines Raumfahrzeugs, das sich im Erdorbit oder an einem Lagrange-Punkt aufhält, und grundsätzlich auch auf der Erde durchgeführt werden. Während und nach der Durchmischung binden die Bindesubstanz 14a und die Detektionssubstanz 20a mit der daran gebundenen Markersubstanz an den Analyten aus der Probe 12a und bilden mit diesem einen Analytkomplex 34a. Nach der Durchmischung wird in dem als ELISA ausgebildeten dargestellten Verfahren das Substrat zu einer Signalerzeugung in das Reaktionsgefäß 26a eingeführt und von der Markersubstanz für einen Farbumschlag gespalten. Nach einer festgelegten, von dem nachzuweisenden Analyten und der Markersubstanz abhängigen Zeitspanne nach der Durchmischung der Probe 12a, der Bindesubstanz 14a und der Detektionssubstanz 20a wird ein Stoppermittel in das Reaktionsgefäß 26a eingeführt, durch das die Umsetzung des Substrats durch die Markersubstanz gestoppt und/oder ein Farbumschlag eines Reaktionsprodukts des gespaltenen Substrats gestoppt wird, um eine Signalsättigung zu vermeiden. Für eine Auslesung werden die magnetischen Inertkörper 18a mittels der Magneteinheit 24a in einer Ecke des Reaktionsgefäßes 26a gesammelt und damit aus einem Detektionsbereich entfernt, um eine Auslesung mittels optischer Methoden zu erleichtern. In einer alternativen Ausgestaltung können die magnetischen Inertkörper 18a mittels der Magneteinheit 24a vollständig aus dem Reaktionsgefäß 26a entfernt werden. In einer weiteren alternativen Ausgestaltung kann eine Positionierung der als magnetischen Inertkörper 18a ausgebildeten Mischungskörper zu einer Auslesung mittels einer künstlich, beispielsweise durch eine Zentrifugation, erzeugten Schwerkraft bewirkt werden. Zu einer Auslesung wird mittels optischer Methoden eine Stärke des Farbumschlags innerhalb des Detektionsbereichs im Reaktionsgefäß 26a bestimmt und mit Referenzmessungen verglichen, um den Analyten quantitativ zu bestimmen.

In den Fig. 2 bis 5 sind vier weitere Ausführungsbeispiele der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Fig. 1 verwiesen wird. Zur Unterscheidung der Ausführungsbeispiele ist der Buchstabe a den Bezugszeichen des Ausführungsbeispiels in der Fig. 1 nachgestellt. In den Ausführungsbeispielen der Fig. 2 bis 5 ist der Buchstabe a durch die Buchstaben b bis e ersetzt.

Fig. 2 zeigt ein Verfahren zur Durchführung einer biochemischen Analyse, bei der zwei unterschiedliche Analyten in einer Probe 12b bestimmt werden. Es werden ein erstes und ein zweites analytspezifisches Paar von Bindesubstanzen 14b, 16b und Detektionssubstanzen 20b, 22b, die zu einer Bestimmung von jeweils zumindest zwei unterschiedlichen Analyten der Probe 12b vorgesehen sind, verwendet. Die Detektionssubstanzen 20b, 22b tragen jeweils eine identische Markersubstanz an sich gebunden, grundsätzlich können die Detektionssubstanzen 20b, 22b jedoch auch unterschiedliche Markersubstanzen an sich gebunden tragen. Die analytspezifischen Bindesubstanzen 14b, 16b sind in einem ersten Verfahrensschritt in einer zumindest weitgehend wasserfreien Form, in der in den Bindesubstanzen14b, 16b ausreichend Restwasser zu einer Erhaltung einer biologischen Funktion enthalten ist, an magnetische Trägerkörper 30b, 32b gebunden in dem Reaktionsgefäß 26b enthalten. In einem weiteren Verfahrensschritt werden in Form einer Lösung die Probe 12b, die Detektionssubstanzen 20b, 22b mit der Markersubstanz und weitere Hilfsstoffe hinzugegeben, wodurch die Bindesubstanzen14b, 16b an den Trägerkörpern 30b, 32b in Lösung gebracht werden und für eine Durchmischung und eine Reaktion mit den Analyten zur Verfügung stehen.

In einem weiteren Verfahrensschritt wird die Durchmischung mittels Mischungskörpern bewirkt, die als die magnetischen Trägerkörper 30b, 32b für die analytspezifischen Bindesubstanzen 14b, 16b ausgebildet sind. Hierzu werden die magnetischen Trägerkörper 30b, 32b mittels einer Magneteinheit 24b auf die bereits zu Fig. 1 beschriebene Weise in Bewegung versetzt. Nach der Durchmischung und einer festgelegten, analytabhängigen Zeitspanne haben die analytspezifischen Bindesubstanzen 14b, 16b und Detektionssubstanzen 20b, 22b mit der Markersubstanz Analytkomplexe 34b, 36b mit den Analyten der Probe 12b ausgebildet. Eine Signalerzeugung durch die Markersubstanz erfolgt wie in dem vorherigen Beispiel. Zu einer Erleichterung einer optischen Auslesung werden die magnetischen Trägerkörper 30b, 32b mit den Analytkomplexen 34b, 36b mittels der Magneteinheit 24b in einem Detektionsbereich an einem Rand des Reaktionsgefäßes 26b positioniert. In einer weiteren alternativen Ausgestaltung kann eine Positionierung der magnetischen Trägerkörper 30b, 32b mit den Analytkomplexen 34b, 36b zu einer Auslesung mittels einer künstlich, beispielsweise durch eine Zentrifugation, erzeugten Schwerkraft bewirkt werden. Die magnetischen Trägerkörper 30b, 32b für die analytspezifischen Bindesubstanzen 14b, 16b sind voneinander bei einer Auslesung unterscheidbar ausgebildet, sodass durch Auslesung des Detektionsbereichs mittels grundsätzlich bekannter optischer Methoden die zwei Analyten qualitativ und quantitativ bestimmt werden können. In einer alternativen Ausgestaltung können die magnetischen Trägerkörper 30b, 32b die Detektionssubstanzen 20b, 22b an sich gebunden tragen.

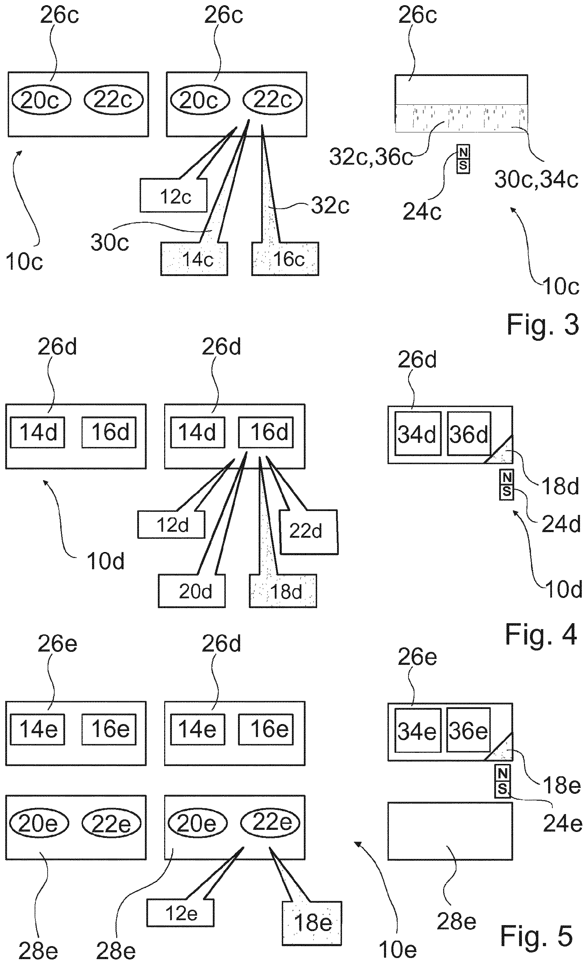

In einem weiteren Ausführungsbeispiel (Fig.3) sind in einem ersten Verfahrensschritt zwei analytspezifische Detektionssubstanzen 20c, 22c in einer weitgehend wasserfreien Form an Oberflächen eines Reaktionsgefäßes 26c gebunden. In einem weiteren Verfahrensschritt werden eine Probe 12c sowie an magnetische Trägerkörper 30c, 32c gebundene Bindesubstanzen 14c, 16c, die Markersubstanz und weiterer Hilfsstoffe wie einem Lösungsmittel für die Detektionssubstanzen 20c, 22c in einer Lösung in das Reaktionsgefäß 26c gegeben. Mittels des Lösungsmittels werden die Detektionssubstanzen 20c, 22c in Lösung gebracht, wodurch sie sich von der Oberfläche des Reaktionsgefäßes 26c ablösen und die analytspezifische Detektionssubstanzen 20c, 22c jeweils an unterschiedliche Analyten binden und ihrerseits die Markersubstanz binden können. Eine Durchmischung wird mittels der magnetischen Trägerkörper 30c, 32c bewirkt, die über eine Magneteinheit 24c in Bewegung versetzt werden. Die Bindesubstanzen 14c, 16c, die Detektionssubstanzen 20c, 22c mit der Markersubstanz sowie die Analyten aus der Probe 12c bilden zwei Arten von Analytkomplexen 34c, 36c aus, die mittels optischer Methoden detektiert werden. Zu einer Erleichterung der Auslesung werden die Trägerkörper 30c, 32c mit den Analytkomplexen 34c, 36c in einem Detektionsbereich an einem Rand des Reaktionsgefäßes 26c gesammelt. In einer weiteren alternativen Ausgestaltung kann eine Positionierung der magnetischen Trägerkörper 30c, 32c mit den Analytkomplexen 34c, 36c zu einer Auslesung mittels einer künstlich, beispielsweise durch eine Zentrifugation, erzeugten Schwerkraft bewirkt werden.

In dem erfindungsgemäßen Ausführungsbeispiel (Fig. 4) werden an einer räumlich vorgegebenen Stelle eines Reaktionsgefäßes 26d unablöslich befestigte Bindesubstanzen 14d, 16d verwendet, die insbesondere durch zugegebene Stoffe nicht abgelöst werden können. Zu einer Durchführung der biochemischen Analyse werden eine Probe 12d, die Detektionssubstanzen 20d, 22d mit daran gebundener Markersubstanz und weitere Hilfsstoffe in Form von Lösungen sowie magnetische Inertkörper 18d in das Reaktionsgefäß 26d gegeben. Eine Durchmischung der Probe 12d der Detektionssubstanzen 20d, 22d mit der Markersubstanz in dem Reaktionsgefäß 26d wird mittels der magnetischen Inertkörper 18d bewirkt, die durch eine Magneteinheit 24d in Bewegung versetzt werden. Während und nach Durchmischung der Probe 12d und der Detektionssubstanzen 20d, 22d mit der Markersubstanz binden die Analyten in der Probe 12d an die analytspezifischen Bindesubstanzen 14d, 16d bzw. die analytspezifischen Detektionssubstanzen 20d, 22d mit der daran gebundenen Markersubstanz und es bilden sich Analytkomplexe 34d, 36d aus den analytspezifischen Bindesubstanzen 14d, 16d, den analytspezifischen Detektionssubstanzen 20d, 22d mit der daran gebundenen Markersubstanz und den jeweiligen Analyten aus, die aufgrund der unablöslich befestigten Bindesubstanzen14d, 16d an einer räumlich vorgegebenen Stelle des Reaktionsgefäßes 26d fest gebunden sind. Die Bindesubstanzen14d, 16d und die Analytkomplexe 34d, 36d sind dabei an räumlich voneinander getrennten Stellen des Reaktionsgefäßes 26d befestigt, sodass eine Auslesung und Unterscheidung der Analyten voneinander erleichtert ist. Zu einer weiteren Erleichterung der Auslesung werden mittels der Magneteinheit 24d die magnetischen Inertkörper 18d an einer von den Analytkomplexen 34d, 36d getrennten Stelle des Reaktionsgefäßes 26d positioniert, sodass sie die Auslesung nicht behindern. In einer weiteren alternativen Ausgestaltung kann eine Positionierung der als magnetische Inertkörper 18d ausgebildeten Mischungskörper zu einer Auslesung mittels einer künstlich, beispielsweise durch eine Zentrifugation, erzeugten Schwerkraft bewirkt werden.

In einem weiteren Ausführungsbeispiel (Fig. 5) umfasst eine Vorrichtung 10e zur Durchführung einer biochemischen Analyse neben einem Reaktionsgefäß 26e, in dem an einer räumlich vorgegebenen Stelle unablöslich befestigte Bindesubstanzen 14e, 16e angeordnet sind, und einer Magneteinheit 24e ein Ladegefäß 28e, in dem während einer Lagerzeit der Vorrichtung 10e zwei analytspezifische Detektionssubstanzen 20e, 22e mit daran gebundener Markersubstanz in einer weitgehend wasserfreien Form an Oberflächen gebunden sind. Bei einer Anwendung des Verfahrens zur Durchführung einer biochemischen Analyse werden in einem Verfahrensschritt eine Probe 12e mit weiteren Hilfsstoffen in Form einer Lösung sowie magnetische Inertkörper 18e in das Ladegefäß 28e eingefüllt, wodurch die Detektionssubstanzen 20e, 22e mit daran gebundener Markersubstanz in Lösung gebracht werden. Die Lösung aus Detektionssubstanzen 20e, 22e, der Probe 12e und magnetischen Inertkörpern 18e wird in einem weiteren Verfahrensschritt in das Reaktionsgefäß 26e transferiert und eine Durchmischung der Detektionssubstanzen 20e, 22e, der Probe 12e und der Bindesubstanzen 14e, 16e wird mittels der magnetischen Inertkörper 18e über die Magneteinheit 24e bewirkt. Eine Ausbildung von Analytkomplexen 34e, 36e erfolgt analog zu einer Ausbildung im Ausführungsbeispiel nach Fig. 4. Eine Auslesung erfolgt auf eine selbe Weise wie in dem in Fig. 4 offenbarten Ausführungsbeispiel. In einer weiteren alternativen Ausgestaltung kann eine Positionierung der als magnetische Inertkörper 18e ausgebildeten Mischungskörper zu einer Auslesung mittels einer künstlich, beispielsweise durch eine Zentrifugation, erzeugten Schwerkraft bewirkt werden.

In den Fig. 2 bis 5 wurden jeweils zwei unterschiedliche analytspezifische Bindesubstanzen 14b-e, 16b-e und Detektionssubstanzen 20b-e, 22b-e verwendet, die zu einer Bestimmung von jeweils zumindest zwei unterschiedlichen Analyten der Proben 12b-e vorgesehen sind. Das genannte Verfahren lässt sich durch Verwendung weiterer analytspezifischer Binde- und Detektionssubstanzen für eine Analyse einer größeren Anzahl Analyten in einer Probe nutzen. In den Fig. 1 bis 5 wurde das Verfahren zur Durchführung einer biochemischen Analyse unter Bedingungen reduzierter Schwerkraft im Weltraum an Bord eines Raumfahrzeugs durchgeführt, grundsätzlich können die Verfahren auch im Erdorbit, auf einem Asteroiden, Mond oder erdfremden Planeten oder auch unter Normalschwerkraft auf der Erde durchgeführt werden.

### Bezugszeichen

- 10: Vorrichtung
- 12: Probe
- 14: Bindesubstanz
- 16: Bindesubstanz
- 18: Inertkörper
- 20: Detektionssubstanz
- 22: Detektionssubstanz
- 24: Magneteinheit
- 26: Reaktionsgefäß
- 28: Ladegefäß
- 30: Trägerkörper
- 32: Trägerkörper
- 34: Analytkomplex
- 36: Analytkomplex

## Patentansprüche

1. Verfahren zur Durchführung einer biochemischen Analyse im Weltraum unter Bedingungen reduzierter Schwerkraft, bei der zumindest ein Analyt in einer Probe (12a-e) qualitativ und/oder quantitativ mittels einer selektiven Bindung eines analytspezifischen Paars aus einer Bindesubstanz (14a-e, 16b-e), die den Analyten fixiert, und einer Detektionssubstanz (20a-e, 22b-e), die an den Analyt bindet und eine Markersubstanz zu einer Markierung des Analyten an sich gebunden trägt oder an sich bindet, bestimmt wird und wobei in einem Verfahrensschritt eine Durchmischung der Probe (12a-e), der Bindesubstanz (14a-e, 16b-e), der Detektionssubstanz (20a-e, 22b-e) und der Markersubstanz in einem Reaktionsgefäß (26a-e) erfolgt, wobei das Verfahren in einer das Reaktionsgefäß (26a-e) und eine Magneteinheit (24a-e) umfassenden Vorrichtung (10a-e) durchgeführt wird,
**dadurch gekennzeichnet, dass**
an einer räumlich vorgegebenen Stelle des Reaktionsgefäßes (26d) die Bindesubstanzen (14d, 16d) als unablöslich befestigte Bindesubstanzen (14d, 16d) verwendet werden, wobei die Durchmischung mittels Mischungskörpern (18a; 18d; 18e, 30b-c, 32b-c) bewirkt wird , wobei die Mischungskörper (18a; 18d; 18e, 30b-c, 32b-c) dazu vorgesehen sind, eine Durchmischung der Probe (12a-e), der Bindesubstanz (14a-e, 16b-e) und der Detektionssubstanz (20a-e, 22b-e) zu bewirken, wobei die Mischungskörper (18a; 18d; 18e, 30b-c, 32b-c) durch eine interne Bewegungsvorrichtung oder durch eine externe Kraft in Bewegung versetzt werden und aufgrund ihrer Bewegung die Probe (12a-e), die Bindesubstanz (14a-e, 16b-e) und die Detektionssubstanz (20a-e, 22b-e) durchmischen, wobei die Durchmischung mittels magnetisch bewegter Mischungskörper (18a; 18d; 18e, 30b-c, 32b-c) bewirkt wird und wobei die Mischungskörper als magnetische Inertkörper ausgebildet sind, wobei die magnetischen Inertkörper magnetische oder magnetisierbare Partikel sind, die frei von Bindungsmöglichkeiten zu Stoffen in der Probe und frei von an sie gebundenen analytspezifischen Bindesubstanzen oder analytspezifische Detektionssubstanzen sind.

2. Verfahren nach Anspruch 1,
**dadurch gekennzeichnet, dass**
eine Positionierung der Mischungskörper (18a; 18d; 18e, 30b-c, 32b-c) zu einer Auslesung mittels einer künstlich erzeugten Schwerkraft bewirkt wird.

3. Verfahren nach einem der vorhergehenden Ansprüche,
**gekennzeichnet durch**
zumindest ein zweites analytspezifisches Paar von Bindesubstanzen (14b-e, 16 b-e) und Detektionssubstanzen (20a-e, 22b-e), das zu einer Bestimmung eines weiteren Analyten vorgesehen ist.

4. Verfahren nach einem der vorhergehenden Ansprüche,
**dadurch gekennzeichnet, dass**
die als magnetisch bewegbare Mischungskörper (18a; 18d; 18e, 30b-c, 32b-c) ausgebildeten Mischungskörper (18a; 18d; 18e, 30b-c, 32b-c) zur Durchmischung der Probe (12a-e) durch ein angelegtes magnetisches Feld in Bewegung versetzt werden.

## Claims

1. Method for performing a biochemical analysis in outer space under conditions of reduced gravity, wherein at least one analyte in a sample (12a-e) is identified qualitatively and/or quantitatively by means of a selective binding of an analyte-specific pair of a binding substance (14a-e, 16b-e) which fixates the analyte and a detection substance (20a-e, 22b-e) which binds to the analyte and either carries bound to it a labelling substance for labelling the analyte or binds to the labelling substance, and wherein, in one method step, a mixing of the sample (12a-e), the binding substance (14a-e, 16b-e), the detection substance (20a-e, 22b-e) and the labelling substance is effected in a reaction vessel (26a-e), the method being carried out in a device (10a-e) comprising the reaction vessel (26a-e) and a magnet unit (24a-e),
**characterised in that**
the binding substances (14d, 16d) are used as non-releasably fixated binding substances (14d, 16d) in a spatially defined place of the reaction vessel (26d), wherein the mixing is effected by means of mixing bodies (18a; 18d; 18e, 30b-c, 32b-c), wherein the mixing bodies (18a; 18d; 18e, 30b-c, 32b-c) are configured to effect a mixing of the sample (12a-e), the binding substance (14a-e, 16b-e) and the detection substance (20a-e, 22b-e), wherein the mixing bodies (18a; 18d; 18e, 30b-c, 32b-c) are brought into motion via an internal motion device or via an external force and, due to their motion, mix the sample (12a-e), the binding substance (14a-e, 16b-e) and the detection substance (20a-e, 22b-e), wherein the mixing is effected by means of magnetically moved mixing bodies (18a; 18d; 18e, 30b-c, 32b-c) and wherein the mixing bodies are embodied as magnetic inert bodies, wherein the magnetic inert bodies are magnetical or magnetisable particles which are free of binding capabilities to substances in the sample and are free from analyte-specific binding substances or analyte-specific detection substances bound to them.

2. Method according to claim 1,
**characterised in that**
a positioning of the mixing bodies (18a; 18d; 18e, 30b-c, 32b-c) for a readout is effected via an artificially generated gravity.

3. Method according to one of the preceding claims,
**characterised by**
at least one second analyte-specific pair of binding substances (14b-e, 16b-e) and detection substances (20a-e, 22b-e), which is configured for an identification of a further analyte.

4. Method according to one of the preceding claims,
**characterised in that**
for mixing the sample (12a-e) the mixing bodies (18a; 18d; 18e, 30b-c, 32b-c), which are implemented as magnetically movable mixing bodies (18a; 18d; 18e, 30b-c, 32b-c), are brought into motion via an applied magnetic field.

## Revendications

1. Procédé de réalisation d'une analyse biochimique en espace sous conditions de gravité réduite, dans lequel au moins un analyte dans un échantillon (12a-e) est identifié qualitativement et/ou quantativement par le biais d'une liaison sélective d'un pair spécifique pour l'analyte, ledit pair étant composé d'un agent liant (14a-e, 16b-e), lequel fixe l'analyte, et en agent capteur (20a-e, 22b-e), lequel se lie à l'analyte, comportant un agent marqueur pour marquer l'analyte en forme lié à lui-même ou liant un agent marqueur à lui-même,
un mélange de l'échantillon (12a-e), de l'agent liant (14a-e, 16b-e), de l'agent capteur (20a-e, 22b-e) et de l'agent marqueur étant effectué dans une étape du procédé dans une cuve de réaction (26a-e), le procédé étant réalisé dans un dispositif (10a-e) comprenant la cuve de réaction (26a-e) aussi qu'une unité magnétique (24a-e),
**caractérisé en ce qu',**
à un lieu spatialement déterminé de la cuve de réaction (26d), les agents liants (14d, 16d) sont utilisés comme des agents liants (14d, 16d) fixés non relâchablement, le mélange étant effectué par le biais des corps mélangeurs (18a ; 18d ; 18e, 30b-c, 32b-c), les corps mélangeurs (18a ; 18d ; 18e, 30b-c, 32b-c) étant prévus à effectuer un mélange de l'échantillon (12a-e), de l'agent liant (14a-e, 16b-e) et de l'agent capteur (20a-e, 22b-e), les corps mélangeurs (18a ; 18d ; 18e, 30b-c, 32b-c) étant mis en mouvement via un dispositif mouvant interne ou par une force externe et mélangeant dû à son mouvement l'échantillon (12a-e), l'agent liant (14a-e, 16b-e) et l'agent capteur (20a-e, 22b-e), le mélange étant effectué par le biais de corps mélangeurs (18a ; 18d ; 18e, 30b-c, 32b-c) mus magnétiquement, les corps mélangeurs (18a ; 18d ; 18e, 30b-c, 32b-c) étant implémentés comme des corps inertes magnétiques, les corps inertes magnétiques constituant des particules magnétiques ou magnétisables, lesquels sont libres des facultés de liaison à des matériaux dans l'échantillon et libres des agents liants spécifiques pour l'analyte ou des agents capteurs spécifiques pour l'analyte, liés auxdits particules.

2. Procédé selon la revendication 1, **caractérisé en ce qu'**un positionnement des corps mélangeurs (18a ; 18d ; 18e, 30b-c, 32b-c) est effectué pour une lecture via une gravité artificiellement générée.

3. Procédé selon l'une quelconque des revendications précédentes, **caractérisé par** au moins un deuxième pair, spécifique pour l'analyte, des agents liants (14b-e, 16b-e) et des agents capteurs (20a-e, 22b-e) prévu pour l'identification d'un autre analyte.

4. Procédé selon l'une quelconque des revendications précédentes, **caractérisé en ce que** les corps mélangeurs (18a ; 18d ; 18e, 30b-c, 32b-c) implémentés comme des corps mélangeurs (18a ; 18d ; 18e, 30b-c, 32b-c) magnétiquement mouvables sont mis en mouvement pour un mélange de l'échantillon (12a-e) par le biais d'un champ magnétique appliqué.
